# EUROPÄISCHE PATENTANMELDUNG

(11) **EP 2 263 643 A2**
(43) Veröffentlichungstag der Anmeldung: **22.12.2010**
(21) Anmeldenummer: 10158194.0
(22) Anmeldetag: 29.03.2010
(51) Int. Cl.: A61K 8/44, A61K 8/49, A61K 8/64, A61K 8/66, A61K 8/67, A61K 8/97, A61Q 19/08

(54) **Antifalten-Kosmetikum enthaltend Pflanzenextrakte**

(30) Priorität: 18.06.2009 DE 102009029813
(71) Anmelder: Henkel AG & Co. KGaA, 40589 Düsseldorf (DE)
(72) Erfinder: Janßen, Frank, 41470, Neuss (DE); Dickhof, Susanne, 41748, Viersen (DE); Emond, Eliane, 40470, Düsseldorf (DE)

(57) **Zusammenfassung**

Kosmetische oder dermatologische topische Zusammensetzungen, enthaltend in einem geeigneten kosmetischen oder dermatologischen Träger - bezogen auf ihr Gewicht - mindestens einen Extrakt aus den Blüten von Lonicera japonica, mindestens einen Extrakt aus den Früchten von Xanthium sibiricum, mindestens einen Extrakt aus den Wurzeln von Cyperus rotundus und 0,001 bis 10 Gew.-% mindestens eines Wirkstoffes aus der Gruppe
- der Monomeren, Oligomeren und Polymeren von Aminosäuren, N-C₂-C₂₄-Acylaminosäuren, den Estern und/oder den physiologisch verträglichen Metallsalzen dieser Substanzen und/oder
- der Extrakte aus der Gruppe, bestehend aus Dill (Peucedanum Graveolens), Korinthe (Johannisbeere), Kardamom (Elettaria cardamomum), schwarzem Rettich, kleiner Stechpalme, Zimt, Hafer (Avena sativa), Kartoffel, Seide, und Asea foetida gum und/oder
- Hyaluronsäure und/oder
- Apfolkernextrakt und/oder
- der Phytohormone und/oder
- der Isoflavonoide und/oder
- der Phytosterole und/oder
- der Triterpenoide und/oder
- der Tocopherole
- der 6,7-disubstituierten 2,2-Dialkylchromanen oder -chromenen der allgemeinen Formeln (II) oder (III), 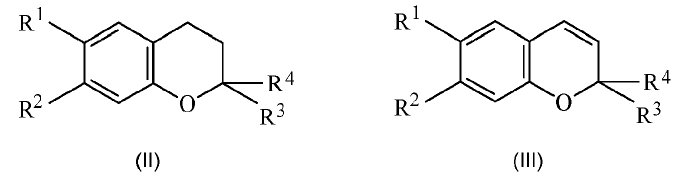 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, insbesondere Lipochroman-6, und/oder
- Ellagsäure und/oder Ellagate und/oder
- der Xanthophylle, insbesondere Astaxanthin, und/oder
- der Superoxiddismutasen und/oder
- Milchsäurebakterien-basierten Fermentationen und/oder
- Ethylhexenoat und seinen Derivaten und/oder
- Methylbutyrat und seinen Derivaten und/oder
- Ethyldecadienoat und seinen Derivaten.

eignen sich hervorragend zum Schutz vor und zur Behandlung von Hautalterungserscheinungen.

## Beschreibung

Die Erfindung betrifft topische kosmetische oder dermatologische Zusammensetzungen zur Hautbehandlung, die der Hautalterung und deren Anzeichen entgegenwirken.

Mittel und Behandlungsmethoden zur Behandlung reifer oder intrinsisch oder extrinsisch gealterter oder lichtgeschädigter Haut, insbesondere zur Antifaltenbehandlung, sind eine bedeutende kosmetische Herausforderung. Der Alterungsprozess ist gekennzeichnet durch einen fortschreitenden Übergang der Hautzellen aus einem proliferativen Status in einen ruhenden, seneszenten Status. Dieser Übergang entspricht der Alterung auf zellulärer Ebene, auf den sich ein Großteil der makroskopischen Alterungseffekte zurückführen lässt. Die Zellzahl im Gewebe verringert sich und kann mangels proliferierender Zellen nicht mehr aufgefüllt werden. Dadurch können keine Reparaturen des umliegenden Gewebes durchgeführt werden, Defekte reichern sich an, wodurch die Haut atrophisch wird und erschlafft. Verstärkt wird dieser Effekt unter anderem auch durch UV-Licht, das die Haut ebenfalls belastet und deren Alterung und Erschlaffung beschleunigt.

Der vorliegenden Erfindung lag die Aufgabe zugrunde, Zubereitungen zur Behandlung und deutlichen Reduzierung der Erscheinungsformen der Hautalterung bereitzustellen, die bislang marktübliche Produkte in der Leistung übertreffen. Es war eine weitere Aufgabe der vorliegenden Erfindung, eine neue, stabile und kosmetisch wirksame Hautpflegezubereitung zur Prophylaxe und Behandlung von Hautalterungserscheinungen, insbesondere Fältchen und Falten zu entwickeln. Insbesondere sollte durch die Hautpflegezubereitung die Hautfeuchtigkeit erhöht werden.

Es wurde nun festgestellt, dass die bekannten Zusammensetzungen in ihren Eigenschaften verbessert werden können, wenn bestimmte an sich bekannte Inhaltsstoffe in diesen Mitteln kombiniert werden.

Gegenstand der vorliegenden Erfindung sind in einer ersten Ausführungsform kosmetische oder dermatologische topische Zusammensetzungen, enthaltend in einem geeigneten kosmetischen oder dermatologischen Träger - bezogen auf ihr Gewicht -
a) mindestens einen Extrakt aus den Blüten von Lonicera japonica,
b) mindestens einen Extrakt aus den Früchten von Xanthium sibiricum,
c) mindestens einen Extrakt aus den Wurzeln von Cyperus rotundus,
d) 0,001 bis 10 Gew.-% mindestens eines Wirkstoffes aus der Gruppe
   - der Monomeren, Oligomeren und Polymeren von Aminosäuren, N-C₂-C₂₄-Acylaminosäuren, den Estern und/oder den physiologisch verträglichen Metallsalzen dieser Substanzen und/oder
   - der Extrakte aus der Gruppe, bestehend aus Dill (Peucedanum Graveolens), Korinthe (Johannisbeere), Kardamom (Elettaria cardamomum), schwarzem Rettich, kleiner Stechpalme, Zimt, Hafer (Avena sativa), Kartoffel, Seide, und Asea foetida gum und/oder
   - Hyaluronsäure und/oder
   - Apfelkernextrakt und/oder
   - der Phytohormone und/oder
   - der Isoflavonoide und/oder
   - der Phytosterole und/oder
   - der Triterpenoide und/oder
   - der Tocopherole
   - der 6,7-disubstituierten 2,2-Dialkylchromanen oder -chromenen der allgemeinen Formeln (II) oder (III), 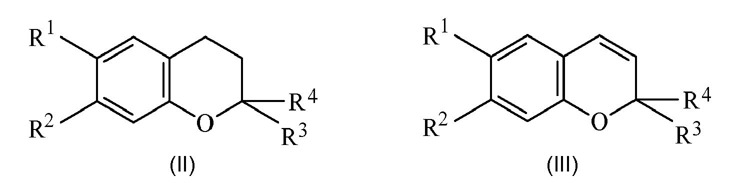 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, insbesondere Lipochroman-6, und/oder
   - Ellagsäure und/oder Ellagate und/oder
   - der Xanthophylle, insbesondere Astaxanthin, und/oder
   - der Superoxiddismutasen und/oder
   - Milchsäurebakterien-basierten Fermentationen und/oder
   - Ethylhexenoat und seinen Derivaten und/oder
   - Methylbutyrat und seinen Derivaten und/oder
   - Ethyldecadienoat und seinen Derivaten.

Die erfindungsgemäßen Zusammensetzungen enthalten mindestens drei Pflanzenextrakte:
- mindestens einen Extrakt aus den Blüten von Lonicera japonica und
- mindestens einen Extrakt aus den Früchten von Xanthium sibiricum und
- mindestens einen Extrakt aus den Wurzeln von Cyperus rotundus.

Ein Extrakt im Sinne der vorliegenden Anmeldung ist ein Stoff oder Stoffgemisch, welches durch Extraktion und teilweises oder völliges Eindampfen der Extraktionslösung gewonnen wurde. Man unterscheidet nach der Beschaffenheit *Trockenextrakte* d.h. bis zur Trockene eingedampfte Extrakte, *Fluidextrakte* d.h. mit Lösungsmitteln so hergestellte Extrakte, dass aus einem Teil Droge höchstens 2 Teile Fluid-Extrakt gewonnen werden, *zähflüssige Extrakte bzw. Dickextrakte,* d.h. Extrakte, bei denen ein Teil des Lösungsmittels verdampft wird.

Die erfindungsgemäß verwendeten Extrakte werden aus den jeweiligen Pflanzenteilen deren Gemischen durch Extraktion vorzugsweise mit organischen Lösemitteln, Wasser oder Gemischen daraus, gewonnen. Bevorzugt geeignete organische Lösemittel sind Ketone (z.B. Aceton), Ether, Ester, Alkohole oder halogenierte Kohlenwasserstoffe. Besonders bevorzugte Extraktionsmittel sind Wasser und/oder Alkohole. Unter den Alkoholen sind dabei (C₁ bis C₆)- Alkohole, wie Ethanol und Isopropanol und zwar sowohl als alleiniges Extraktionsmittel als auch in Mischung mit Wasser, bevorzugt. Besonders bevorzugte Extraktionsmittel sind Wasser, Ethanol, 2-Propanol, 1,2-Propylenglycol, 1,3-Butylenglycol, ganz besonders bevorzugt sind Wasser, Ethanol, 2-Propanol und 1,2-Propylenglycol sowie Mischungen hiervon, z. B. eine Mischung 1,2-Propylenglycol/ Wasser im Verhältnis 4:1.

Die Extraktion wird bevorzugt bei einer Temperatur von 25°C bis 90°C durchgeführt.

Besonders bevorzugte erfindungsgemäße kosmetische Mittel sind **dadurch gekennzeichnet, dass** der Extrakt mindestens ein polares Lösungsmittel, ausgewählt aus Ethanol, 1-Propanol, 2-Propanol, 1-Butanol, 2-Butanol, Ethylenglycol, 1,2-Propylenglycol, 1,3-Butylenglycol, Glycerin und Wasser, sowie Mischungen hiervon enthält.

Je nach Wahl der Extraktionsmittel kann es bevorzugt sein, den Extrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Ölen. Bevorzugte Lösungsvermittler sind ethoxylierte Mono-, Di- und Triglyceride von C8-22-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglycolcapryl-/caprinsäureglyceride, Polyoxyethylenglycerinmonolaurat und Polyoxyethylenglycolkokosfettsäureglyceride. Besonders bevorzugt ist Olivenölethoxylat (INCI-Bezeichnung: PEG-10 Olive Glycerides).

Die Trockenmasse des Extraktes ist abhängig von der Molmasse und der Löslichkeit der dispergierten Inhaltsstoffe und beträgt in der Regel, jeweils bezogen auf das Gewicht des Extraktes, 1 bis 80 Gew.-%. Bevorzugt beträgt die Trockenmasse 15 bis 50 Gew.-% und besonders bevorzugt 20 bis 35 Gew.-%. Bei einem Molekulargewicht der Inhaltsstoffe über 100.000 Dalton kann eine Trockenmasse von 1 bis 20 Gew.-% bevorzugt und eine Trockenmasse von 1 bis 10 Gew.-% besonders bevorzugt sein. Besonders bevorzugte kosmetische Mittel sind dadurch gekennzeichnet, dass die Trockenmasse des Extraktes 5-80 Gew.-%, bezogen auf das Gewicht des Extraktes, beträgt.

Die erfindungsgemäßen Zusammensetzungen enthalten mindestens einen Extrakt aus den Blüten von Lonicera japonica ("Japanisches Geißblatt"). Erfindungsgemäß bevorzugte Zusammensetzungen sind **dadurch gekennzeichnet, dass** sie - bezogen auf ihr Gewicht - 0,001 bis 10 Gew.-%, vorzugsweise 0,002 bis 9 Gew.-%, weiter bevorzugt 0,005 bis 8 Gew.-%, noch weiter bevorzugt 0,01 bis 7 Gew.-% und insbesondere 0,05 bis 6 Gew.-% Extrakt(e) aus den Blüten von Lonicera japonica enthalten.

Die erfindungsgemäßen Zusammensetzungen enthalten mindestens einen Extrakt aus den Früchten von Xanthium sibiricum ("Spitzkletten"). Erfindungsgemäß bevorzugte Zusammensetzungen sind **dadurch gekennzeichnet, dass** sie - bezogen auf ihr Gewicht - 0,001 bis 10 Gew.-%, vorzugsweise 0,002 bis 9 Gew.-%, weiter bevorzugt 0,005 bis 8 Gew.-%, noch weiter bevorzugt 0,01 bis 7 Gew.-% und insbesondere 0,05 bis 6 Gew.-% Extrakt(e) aus den Früchten von Xanthium sibiricum enthalten.

Die erfindungsgemäßen Zusammensetzungen enthalten mindestens einen Extrakt aus den Wurzeln von Cyperus rotundus (Nagarmustaka). Erfindungsgemäß bevorzugte Zusammensetzungen sind **dadurch gekennzeichnet, dass** sie - bezogen auf ihr Gewicht - 0,001 bis 10 Gew.-%, vorzugsweise 0,002 bis 9 Gew.-%, weiter bevorzugt 0,005 bis 8 Gew.-%, noch weiter bevorzugt 0,01 bis 7 Gew.-% und insbesondere 0,05 bis 6 Gew.-% Extrakt(e) aus den Wurzeln von Cyperus rotundus enthalten.

Ganz besonders bevorzugte erfindungsgemäße Zusammensetzungen enthalten die drei Extrakte (mindestens einen Extrakt aus den Blüten von Lonicera japonica und mindestens einen Extrakt aus den Früchten von Xanthium sibiricum und mindestens einen Extrakt aus den Wurzeln von Cyperus rotundus) in einer Gesamtmenge von - bezogen auf das Gewicht der Zusammensetzung - 0,001 bis 15 Gew.-%, vorzugsweise 0,005 bis 12 Gew.-%, weiter bevorzugt 0,01 bis 10 Gew.-%, noch weiter bevorzugt 0,05 bis 7 Gew.-% und insbesondere 0,15 bis 6 Gew.-%.

Die erfindungsgemäßen Mittel enthalten zusätzlich zu den genannten Pflanzenextrakten mindestens einen Wirkstoff aus der Gruppe der Rohstoffe, die die Kollagen- und/oder Glycoaminoglucansynthese, Epidermisdifferenzierung und/oder die Elastinintegrität verbessern. Als ein bevorzugter Inhaltsstoff aus dieser Gruppe haben sich Monomere, Oligomere und Polymere von Aminosäuren, N-C₂-C₂₄-Acylaminosäuren, den Estern und/oder den physiologisch verträglichen Metallsalzen dieser Substanzen bewährt.

Bevorzugte erfindungsgemäße Zusammensetzungen sind **dadurch gekennzeichnet, dass** die Monomeren von Aminosäuren, N-C₂-C₂₄-Acylaminosäuren und/oder den physiologisch verträglichen Metallsalzen dieser Substanzen ausgewählt sind aus Alanin, Arginin, Asparagin, Asparaginsäure, Canavanin, Citrullin, Cystein, Cystin, Desmosin, Dipalmitoylhydroxyprolin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat.

Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.

Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.

Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2-15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin 1 (TSP-1) zu binden und damit aktives TGF-β (*tissue growth factor*), der die Synthese von Collagen in dermalen Fibroblasten induziert, freizusetzen. Derartige Aminosäureoligomere können als Wirkstoffe gegen die Hautalterung verwendet werden.

Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produkts Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn N-Elaidoyl-Lys-Phe-Lys und N-Acetyl-Arg-Lys-Arg-NH₂.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im Folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.

Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).

Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.

Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.

Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.

Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (Glistin von Exsymol).

Erfindungsgemäß besonders bevorzugt ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.

Die Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, besonders bevorzugt Sojaproteinhydrolysate mit einem mittleren Molekulargewicht im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine^{®} von Coletica erhältlich. mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 - 18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind.

Ein weiteres, erfindungsgemäß besonders bevorzugtes Sojaproteinhydrolysat ist ein mit Kokosfettsäuren N-acyliertes und/oder verestertes Sojaproteinhydrolysat in Form des Kaliumsalzes, das unter der Handelsbezeichnung Coccopolipeptide di Soja von der Firma Sinerga erhältlich ist. Weiterhin erfindungsgemäß bevorzugt sind Keratinhydrolysate, insbesondere Wollkeratinhydrolysate. Ein besonders bevorzugtes Wollkeratinhydrolysat ist unter der Bezeichnung Keratec Pep von der Firma Croda erhältlich. Keratec Pep weist eine kleinere Molekulargewichtsfraktion mit einem durchschnittlichen Molekulargewicht von 150 Dalton und eine größere Molekulargewichtsfraktion mit einem durchschnittlichen Molekulargewicht von 1265 Dalton auf. Weiterhin erfindungsgemäß bevorzugt sind Conchiolinhydrolysate, insbesondere solche, die unter den Bezeichnungen Pearl Protein Extract und Pearl Protein Extract BG von der Firma Maruzen erhältlich sind. Conchiolin ist ein komplexes Protein, das aus dem äußeren Epithelium von Mollusken, insbesondere von Perlmuscheln und diversen Schneckenarten, erzeugt wird und das durch Einlagerung von Calciumcarbonat-Kristallen die sehr stabile Schale dieser Mollusken bildet. Proteinhydrolysate können naturgemäß auch monomere Aminosäuren und Oligopeptide enthalten; ihre Zusammensetzung ist normalerweise nicht definiert.

Ebenfalls bevorzugt ist der Einsatz von Acylderivaten der Proteinhydrolysate, z. B. in Form ihrer Fettsäure-Kondensationsprodukte.

Zusammenfassend sind erfindungsgemäß bevorzugte Zusammensetzungen dadurch gekennzeichnet, dass die Oligomeren von Aminosäuren, N-C₂-C₂₄-Acylaminosäuren und/oder den physiologisch verträglichen Metallsalzen dieser Substanzen ausgewählt sind aus Di-, Tri-, Tetra-, Penta-, Hexa- oder Pentadecapeptiden, die N-acyliert und/oder verestert sein können.

Ganz besonders bevorzugte erfindungsgemäße Zusammensetzungen sind dadurch gekennzeichnet, dass das Oligopeptid ausgewählt ist aus Tyr-Arg und seinen N-acylierten Derivaten, insbesondere N-Acetyl-Tyr-Arg-hexyldecylester, Gly-His-Lys und seinen N-acylierten Derivaten, insbesondere N-Palmitoyl-Gly-His-Lys, Gly-His-Arg, und seinen N-acylierten Derivaten, insbesondere N-Myristoyl-Gly-His-Arg, Lys-Val-Lys und seinen N-acylierten Derivaten, insbesondere Palmitoyl-Lys-Val-Lys, Val-Tyr-Val, Gly-Gln-Pro-Arg (Rigin), Rigin-Analoga und Rigin-Derivaten, insbesondere N-Palmitoyl-Gly-Gln-Pro-Arg, Lys-Thr-Thr-Lys-Ser und seinen N-acylierten Derivaten, insbesondere N-Palmitoyl-Lys-Thr-Thr-Lys-Ser, Val-Gly-Val-Ala-Pro-Gly und seinen N-acylierten Derivaten, insbesondere N-Palmitoyl-Val-Gly-Val-Ala-Pro-Gly, Acetyl-Hexapeptide-3, Hexapeptide-4, Hexapeptide-5, Myristoyl Hexapeptide-5, Myristoyl Hexapeptide-6, Hexapeptide-8, Hexapeptide-9, Hexapeptide-10, L-Glutamylaminoethyl-indol, sowie Kombinationen dieser Substanzen, insbesondere Kombinationen aus N-Palmitoyl-Gly-His-Lys und N-Palmitoyl-Gly-Gln-Pro-Arg. Ebenfalls bevorzugt sind Zusammensetzungen, bei denen die Polymeren von Aminosäuren, N-C₂-C₂₄-Acylaminosäuren und/oder den physiologisch verträglichen Metallsalzen dieser Substanzen ausgewählt sind aus tierischen und pflanzlichen Proteinhydrolysaten, tierischen und pflanzlichen Proteinen und DNA-Reparaturenzymen. Hier sind ganz besonders bevorzugte erfindungsgemäße Zusammensetzungen **dadurch gekennzeichnet, dass** sie zusätzlich mindestens ein DNA-Reparaturenzym, vorzugsweise Photolyase und/oder T4 Endonuclease V und/oder 8-Oxoguanin-glycosylase und/oder deren Mischungen enthalten.

Die DNA-Reparaturenzyme können sowohl in Liposomen verkapselt als auch frei, das heißt unverkapselt, vorliegen. Die Liposomenverkapselung kann bevorzugt mit Phospholipiden, besonders bevorzugt mit Lecithin, erfolgen. Erfindungsgemäß besonders bevorzugt ist der Einsatz von mindestens einem liposomenverkapselten DNA-Reparaturenzym. Liposomenverkapselte Photolyase ist im Handel z. B. unter der Produktbezeichnung Photosomes™ (INCI-Bezeichnung: Aqua, Lecithin, Plankton Extract) liposomenverkapselte T4N5 z. B. unter der Bezeichnung Ultrasomes™ (INCI-Bezeichnung: Aqua, Lecithin, Micrococcus Lysate) von der Firma AGI Dermatics, USA, erhältlich. Da sich bei längerer Lagerung sowohl des Handelsprodukts als auch der erfindungsgemäßen Zusammensetzungen zwischen der verkapselten wässrigen, das Enzym enthaltenden Phase und der nicht-verkapselten, äußeren wässrigen Phase ein Gleichgewicht einstellt, liegt ein Teil der DNA-Reparaturenzyme unverkapselt vor.

In den erfindungsgemäßen Zusammensetzungen sind die Handelsprodukte Photosomes™ oder Ultrasomes™ bevorzugt in Mengen von 0,1 - 10 Gew.-%, besonders bevorzugt 0,5 - 5,0 Gew.-% und außerordentlich bevorzugt 1,0 -4,0 Gew.-%, bezogen auf das gesamte Mittel, enthalten. Bevorzugte erfindungsgemäße Zusammensetzungen sind **dadurch gekennzeichnet, dass** mindestens ein DNA-Reparaturenzym in Gesamtmengen von 0,00001 - 1 Gew.-%, bevorzugt 0,0001 - 0,1 Gew.-%, besonders bevorzugt 0,001 - 0,01 - 0,05 Gew.-% und außerordentlich bevorzugt 0,002 - 0,005 Gew.-%, enthalten ist.

Ganz besonders bevorzugte erfindungsgemäße Hautbehandlungsmittel sind dadurch gekennzeichnet, dass sie das/die DNA-Reparaturenzym(e) - bezogen auf das Gewicht des Hautbehandlungsmittels - in Mengen von 0,00001 - 1 Gew.-%, bevorzugt 0,0001 - 0,1 Gew.-%, besonders bevorzugt 0,001 -0,05 Gew.-% und außerordentlich bevorzugt 0,002 - 0,005 Gew.-%, enthalten. Anstelle der vorstehend genannten Verbindungen oder in Ergänzung zu ihnen können die erfindungsgemäßen Mittel auch Wirkstoffe enthalten, die die enzymatische Aktivität oder die Expression der L-Isoform der Lysyloxidase (LOXL) stimulieren.

Mit dem Begriff "LOXL" oder "hLOXL" ist die L-Isoform des humanen Proteins Lysyloxidase LOXL gemeint. Mit dem Begriff "LOX" oder "hLOX" ist die Anfangs-Isoform ("Pisoform initiale" des humanen Proteins Lysyloxidase LOX gemeint. Mit "Stimulieren der Expression der Isoform der Lysyloxidase LOXL" ist die Stimulierung der Expression des Gens, das für LOXL kodiert, oder von dessen Promotor gemeint, und insbesondere ist die Stimulierung der Synthese der messenger RNA, die für LOXL kodiert, und ebenfalls die Stimulierung der Synthese von LOXL, das aus dieser messenger RNA resultiert, gemeint. Mit "Stimulieren der Expression von Elastin der Isoform der Lysyloxidase LOXL" ist die Stimulierung der Expression des Gens, das für das Protein Elastin kodiert, oder von dessen Promotor gemeint, und insbesondere ist die Stimulierung der Synthese der messenger RNA, die für das Protein Elastin kodiert, und ebenfalls die Stimulierung der Synthese des Proteins Elastin oder seines Vorläufers, Tropoelastin, aus dieser messenger RNA resultiert, gemeint.

Wirkstoffe sind dann als wirksam anzusehen, wenn sie eine Aktivierung oder einen 1,5-fachen Anstieg der Expression und/oder der Aktivität von LOXL in einem Modell erzielen, das mindestens einen Zelltyp umfasst, der eine Expression und/oder Aktivität von LOXL über den Kontakt mit diesen Wirkstoffen aufweist, in Bezug auf den Expressionslevel und/oder Aktivitätslevel von LOXL in einem Kontrollmodell (ohne Kontakt mit den Wirkstoffen).

Vorteilhafterweise ist die Expression von LOXL entweder die Expression einer Nukleotidsequenz, die für LOXL kodiert, oder die Expression einer Aminosäuresequenz, die eine Teilsequenz des Proteins LOXL darstellt.

Vorteilhafterweise wird die Wirksubstanz ausgewählt aus der Gruppe
- der Extrakte aus der Gruppe, bestehend aus Dill (Peucedanum Graveolens), Korinthe (Johannisbeere), Kardamom (Elettaria cardamomum), schwarzem Rettich, kleiner Stechpalme, Zimt, Hafer (Avena sativa), Kartoffel, Seide, und Asea foetida gum und/oder
- Milchsäurebakterien-basierten Fermentationen und/oder
- Ethylhexenoat und seinen Derivaten und/oder
- Methylbutyrat und seinen Derivaten und/oder
- Ethyldecadienoat und seinen Derivaten.

Anstelle der vorstehend genannten Verbindungen oder in Ergänzung zu ihnen können die erfindungsgemäßen Mittel auch Hyaluronsäure(Hyaluronan) enthalten. Hyaluronsäure ist ein Glycosaminoglycan. Hyaluronsäure ist eine hochmolekulare Verbindung mit Molmassen zwischen 50000 und mehreren Millionen. Grundbaustein der Hyaluronsäure ist ein aus D-Glucuronsäure und N-Acetyl-D-glucosamin in β-(1, 3)-glycosidischer Bindung aufgebautes Aminodisaccharid, das mit der nächsten Einheit β-(1, 4)-glycosidisch verbunden ist.

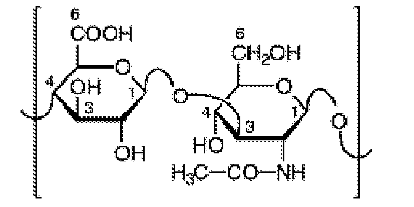

Die unverzweigte Kette der Hyaluronsäure besteht aus 2000-10000 solcher Einheiten. Hyaluronsäure-Ketten sind in Lösung helical angeordnet.

Anstelle der vorstehend genannten Verbindungen oder in Ergänzung zu ihnen können die erfindungsgemäßen Mittel auch Extrakte aus Apfelkernen enthalten.

Ein erfindungsgemäß besonders bevorzugter Apfelkernextrakt ist das Handelsprodukt Ederline des Herstellers Seporga. Ederline enthält Phytohormone, Isoflavonoide, Phytosterole, Triterpenoide, Tocopherole und natürliche Wachse. In vitro-Tests mit kultivierten Hautzellen zeigen laut Herstellerangaben Effekte auf die Synthese von Kollagen Typ I und Kollagen Typ III sowie auf Fibronectin. Desweiteren wurde in Probandenstudien eine positive Wirkung von Ederline auf das Hautrelief gemessen. Außerdem machen die Herstellerangaben antioxidative und antiinflammatorische Wirkungen geltend. Das Produkt Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich.

Erfindungsgemäß geeignete Mengen an Ederline sind 0,1 - 10 Gew.-%, bevorzugt 1 - 8 Gew.-% und besonders bevorzugt 3 - 5 Gew.-%, jeweils bezogen auf die gesamte Zusammensetzung. Bezogen auf den Gehalt an aktiven Wirkstoffen, werden Apfelkernextrakte erfindungsgemäß in Mengen von 0,001 - 2 Gew.-%, bevorzugt 0,01 - 1,6 Gew.-% und besonders bevorzugt 0,03 - 1 Gew.-%, jeweils bezogen auf die gesamte Zusammensetzung, eingesetzt.

Anstelle der vorstehend genannten Verbindungen oder in Ergänzung zu ihnen können die erfindungsgemäßen Mittel auch Phytohormone enthalten. Unter Phytohormonen (Pflanzenwuchsstoffen) werden im Rahmen der vorliegenden Erfindung Pflanzenwuchsstoffe aus den Gruppen der Derivate der 1*H*-Indol-3-ylessigsäure, Gibberelline, Cytokinine und Abscisinsäure verstanden. In die erste Gruppe gehören z. B. die 2-Naphthyloxyessigsäure, Benzoesäure-Derivate, Phenoxycarbonsäure-Derivate usw.

Anstelle der vorstehend genannten Verbindungen oder in Ergänzung zu ihnen können die erfindungsgemäßen Mittel auch Isoflavonoide enthalten. Isoflavonoide sind zu den Flavonoiden zählende, gelegentlich auch als Isoflavone bezeichnete Gruppe von meist gelblich gefärbten Pflanzenfarbstoffen, die sich von Isoflavon ableiten. Der Grundkörper Isoflavon (3-Phenylchromon, 3-Phenyl-4*H*-1-benzopyran-4-on) kommt in Kleearten vor.

Einige bekanntere Isoflavone sind *Daidzein* (4',7-Dihydroxy-loflavon), *Genistein* (4',5,7-Trihydroxy-Isoflavion); *Prunetin* (4',5-Dihydroxy-7-methoxy-Isoflavon); *Biochanin A* (5,7-Dihydroxy-4'-methoxy-Isoflanon); *Orobol* (3',4',5,7-Tetrahydroxy-Isoflavon); *Santal* (3',4',5-Trihydroxy-7-methoxy-Isoflavon).

Anstelle der vorstehend genannten Verbindungen oder in Ergänzung zu ihnen können die erfindungsgemäßen Mittel auch Phytosterole enthalten. Phytosterole (Phytosterine) sind Sterole aus Pflanzen und Hefen (Mykosterole). Sie unterscheiden sich von Sterolen tierischen Ursprungs durch eine Doppelbindung an C-22 und C₁- od. C₂-Substituenten an C-24. Die häufigsten pflanzlichen Sterole sind Stigmasterol, β-Sitosterol und Campesterol.

Anstelle der vorstehend genannten Verbindungen oder in Ergänzung zu ihnen können die erfindungsgemäßen Mittel auch Triterpenoide enthalten. Triterpenoide (Triterpe) sind aus 6 Isopren-Einheiten aufgebaute Naturstoffe mit 30 Kohlenstoff-Atomen, die zur Gruppe der Terpene gehören. Zur Zeit sind rund 5000 Triterpene bekannt. Das einfachste Triterpen ist das acyclische Squalen, das auch die Muttersubstanz aller weiteren Triterpene ist. Monocyclische, bicyclische und tricyclische Triterpene sind sehr selten. Sie entstehen infolge einer gestörten Squalen-Cyclisierung, so z. B. das tricyclische Ambrein. Die Mehrzahl der Triterpene ist tetracyclisch bzw. pentacyclisch.

Anstelle der vorstehend genannten Verbindungen oder in Ergänzung zu ihnen können die erfindungsgemäßen Mittel auch Tocopherole enthalten. Tocopherole ist die Bezeichnung für in 2-Stellung mit einem 4,8,12-Trimethyltridecyl-Rest substituierte Chroman-6-ole (3,4-Dihydro-2*H*-1-benzopyran-6-ole).

Als eine weitere besonders geeignete Verbindung können die erfindungsgemäßen Mittel 6,7-disubstituierte 2,2-Dialkylchromane oder -chromene der allgemeinen Formeln (II) oder (III), 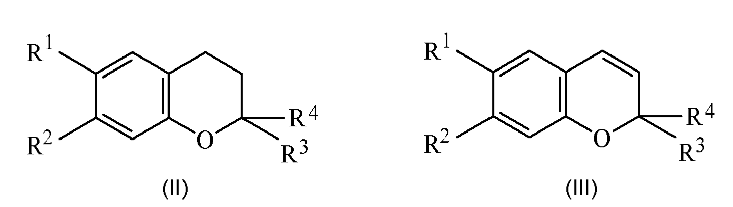 enthalten, wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen. Die Substituenten R¹ und R² sind unabhängig voneinander ausgewählt aus einer OH-Gruppe, einer Methoxy-Gruppe und einer CF₃CH₂O-Gruppe. In einer bevorzugten Ausführungsform sind R¹ und R² unabhängig voneinander ausgewählt aus einer OH-Gruppe und einer Methoxy-Gruppe. In einer besonders bevorzugten Ausführungsform stellen R¹ eine OH-Gruppe und R² eine Methoxy-Gruppe dar.

Die Substituenten R³ und R⁴ stellen unabhängig voneinander eine C₁-C₄-Alkylgruppe dar. Unter C₁-C₄-Alkylgruppe ist dabei erfindungsgemäß eine Methyl-, Ethyl-, n-Propyl-, Isopropyl-, n-Butyl-, Isobutyl- beziehungsweise 2-Methylpropyl-, sec-Butyl- beziehungsweise 1-Methylpropyl- oder eine tert.-Butyl-Gruppe zu verstehen. In einer bevorzugten Ausführungsform sind R³ und R⁴ unabhängig voneinander ausgewählt aus einer Methyl-, Ethyl-, n-Propyl-, Isopropyl- und einer n-Butyl-Gruppe. Besonders bevorzugt stellen R³ und R⁴ unabhängig voneinander eine Methyl- oder Ethylgruppe dar. Außerordentlich bevorzugt ist, dass R³ und R⁴ identisch sind.

Erfindungsgemäß bevorzugt verwendet werden die 6,7-disubstituierten 2,2-Dialkylchromane. Ein erfindungsgemäß außerordentlich bevorzugt verwendeter Wirkstoff ist 2,2-Dimethyl-6-hydroxy-7-methoxy-chroman mit der systematischen Bezeichnung 3,4-Dihydro-7-methoxy-2,2-dimethyl-2H-1-benzopyran-6-ol und der INCI-Bezeichnung Dimethylmethoxy Chromanol. Die Substanz ist unter dem Handelsnamen Lipochroman-6 von der Firma Lipotec S.A. erhältlich.

Die 6,7-disubstituierten 2,2-Dialkylchromane oder -chromene der allgemeinen Formeln (II) oder (III) werden vorzugsweise in Mengen von 0,001 - 5 Gew.-%, bevorzugt 0,005 - 2 Gew.-% und besonders bevorzugt 0,01 - 0,1 Gew.-%, jeweils bezogen auf die gesamte Zusammensetzung, eingesetzt. Zusammenfassend sind erfindungsgemäße Hautbehandlungsmittel bevorzugt, die - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% 6,7-disubstituierte 2,2-Dialkylchromane oder -chromene der allgemeinen Formeln (II) oder (III) wie vorstehend definiert, enthalten, wobei bevorzugte Mittel - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Lipochroman-6 enthalten. Weitere erfindungsgemäß bevorzugte Zusammensetzungen enthalten - bezogen auf ihr Gewicht - 3,4-Dihydro-7-methoxy-2,2-dimethyl-2H-1-benzopyran-6-ol in einer Gesamtmenge von 0,001 - 1 Gew.-%, bevorzugt 0,005 - 0,5 Gew.-%, besonders bevorzugt 0,01 - 0,2 Gew.-%, außerordentlich bevorzugt 0,05 - 0,1 Gew.-%.

Als eine weitere besonders geeignete Verbindung können die erfindungsgemäßen Mittel mindestens einen Stoff aus der Gruppe der Ellagsäure und/oder Ellagate enthalten. Ellagsäure (2,3,7,8-Tetrahydroxy[1]benzopyrano-[5,4,3-*cde*][1]benzopyran-5,10-dion 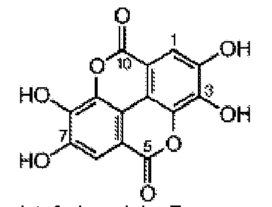 ist frei und in Form von Glycosiden und/oder Methylethern im Pflanzenreich weit verbreitet, besonders in Galläpfeln, Blattgallen, auch in den Blättern von *Eucalyptus-Arten,* in Eichen und Euphorbien.

Bevorzugte erfindungsgemäße Zusammensetzungen sind **dadurch gekennzeichnet, dass** sie - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Ellagsäure (2,3,7,8-Tetrahydroxy[1]benzopyrano-[5,4,3-cde][1]benzopyran-5,10-dion) und/oder Ellagate enthalten.

Als eine weitere besonders geeignete Verbindung können die erfindungsgemäßen Mittel mindestens einen Stoff aus der Gruppe der Xanthophylle, insbesondere Astaxanthin, enthalten.

Xanthophylle ist der Gruppenname für Hydroxy-, Epoxy- und Oxo-Derivate von Carotinoiden (= Tetraterpenen mit einem C₄₀-Grundgerüst). Beispiele für erfindungsgemäß geeignete Verbindungen sind z. B. Canthaxanthin, Citranaxanthin, Flavoxanthin, Rhodoxanthin, Rubixanthin, Zeaxanthin, Lutein, Cryptoxanthin, Violaxanthin und insbesondere Astaxanthin. Die natürlich vorkommenden Xanthophylle sind meist *all*-*trans*-Verbindungen.

Erfindungsgemäß bevorzugte Zusammensetzungen enthalten - bezogen auf ihr Gewicht - 0,001 bis 10 Gew.-%, vorzugsweise 0,005 bis 7,5 Gew.-%, weiter bevorzugt 0,01 bis 5 Gew.-% und insbesondere 0,02 bis 1 Gew.-% Xanthophylle, vorzugsweise Canthaxanthin, Citranaxanthin, Flavoxanthin, Rhodoxanthin, Rubixanthin, Zeaxanthin und ganz besonders bevorzugt Astaxanthin.

Als eine weitere besonders geeignete Verbindung können die erfindungsgemäßen Mittel mindestens einen Stoff aus der Gruppe der Superoxiddismutasen enthalten. Erfindungsgemäß bevorzugte Zusammensetzungen enthalten- bezogen auf ihr Gewicht - 0,001 bis 10 Gew.-%, vorzugsweise 0,005 bis 7,5 Gew.-%, weiter bevorzugt 0,01 bis 5 Gew.-% und insbesondere 0,02 bis 2 Gew.-% Superoxiddismutase(n).

In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C und E und den Estern der vorgenannten Substanzen. Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente erfindungsgemäß besonders bevorzugt sind Vitamin A-Säure und deren Ester, Vitamin A-Aldehyd und Vitamin A-Alkohol sowie dessen Ester, wie Retinylpalmitat und Retinylacetat. Erfindungsgemäß besonders bevorzugte Zusammensetzungen sind **dadurch gekennzeichnet, dass** sie mindestens ein Vitamin, Provitamin oder eine als Vitaminvorstufe bezeichnete Verbindung aus den Vitamingruppe A oder mindestens einen Ester hiervon in Gesamtmengen von 0,001 - 2 Gew.-%, bevorzugt 0,05 - 0,05 - 1 Gew.-%, bezogen auf die gesamte Zusammensetzung, enthalten.

Zur Vitamin B-Gruppe oder zu dem Vitamin B-Komplex gehören unter anderem
- Vitamin B₁, Trivialname Thiamin, chemische Bezeichnung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,0005 bis 0,1 - 1 Gew.-%, bezogen auf die gesamte erfindungsgemäße Zusammensetzung, eingesetzt.
- Vitamin B₂, Trivialname Riboflavin, chemische Bezeichnung 7,8-Dimethyl-10-(1-D-ribityl)-benzo[g]pteridin-2,4(3*H*,10*H*)-dion. Bevorzugt werden Riboflavin oder seine Derivate in Mengen von 0,0005 bis 0,1 - 1 Gew.-%, bezogen auf die gesamte erfindungsgemäße Zusammensetzung, eingesetzt.
- Vitamin B₃- Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,0005 bis 0,1 - 1 Gew.-%, bezogen auf die gesamte erfindungsgemäße Zusammensetzung, enthalten ist.
- Vitamin B₅ (Pantothensäure und Panthenol). Bevorzugt wird Panthenol eingesetzt. Erfindungsgemäß bevorzugte Derivate des Panthenols sind insbesondere die Ester und Ether des Panthenols sowie kationisch derivatisierte Panthenole. In einer weiteren bevorzugten Ausführungsform der Erfindung werden an Stelle von sowie zusätzlich zu Pantothensäure oder Panthenol auch Derivate des 2-Furanon mit der allgemeinen Strukturformel (VIT-I) eingesetzt. 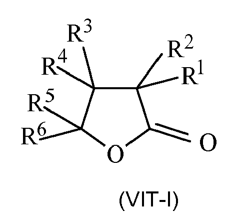 Besonders bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
   Erfindungsgemäß besonders bevorzugte Zusammensetzungen sind **dadurch gekennzeichnet, dass** sie mindestens eine der genannten Verbindungen des Vitamin B₅-Typs sowie der 2-Furanonderivate in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Erfindungsgemäß besonders bevorzugte Zusammensetzungen sind **dadurch gekennzeichnet, dass** sie mindestens eine Vitamin B₆-Komponente in einer Gesamtmenge von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Erfindungsgemäß besonders bevorzugte Zusammensetzungen sind **dadurch gekennzeichnet, dass** sie mindestens eine Komponente, ausgewählt aus Biotin und den Biotinestern, in einer Gesamtmenge von 0,0001 bis 1,0 Gew.-%, insbesondere 0,001 bis 0,01 Gew.-%, enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind **dadurch gekennzeichnet, dass** sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind **dadurch gekennzeichnet, dass** sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und aus Pantolacton. Bevorzugte Vitamine, Provitamine und Vitaminvorstufen der Gruppe C und deren Ester sind Vitamin C (Ascorbinsäure) und die Derivate Ascorbylpalmitat, -stearat, -dipalmitat, -acetat, Magnesiumascorbylphosphat, Natriumascorbylphosphat, Natrium- und Magnesiumascorbat, Dinatriumascorbylphosphat und -sulfat, Kaliumascorbyltocopherylphosphat, Chitosanascorbat oder Ascorbylglucosid. Die Kombination mit Tocopherolen kann ebenfalls bevorzugt sein. Erfindungsgemäß besonders bevorzugte Zusammensetzungen sind **dadurch gekennzeichnet, dass** sie mindestens eine der genannten Verbindungen des Vitamin C-Typs in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Erfindungsgemäß besonders bevorzugte Zusammensetzungen sind **dadurch gekennzeichnet, dass** sie mindestens eine Substanz, ausgewählt aus Tocopherol und seinen Derivaten, in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Vitamin A-palmitat (Retinylpalmitat), Pantolacton, Nicotinsäureamid, Pyridoxin, Pyridoxamin, Pyridoxal, Biotin, Ascorbylpalmitat, Ascorbylacetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

Zusammenfassend sind erfindungsgemäße Zusammensetzungen bevorzugt, die zusätzlich - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel Panthenol ((±)-2,4-Dihydroxy-N-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Weitere bevorzugte erfindungsgemäße Zusammensetzungen sind **dadurch gekennzeichnet, dass** zur Verbesserung der Hautfeuchtigkeit unter Verzicht auf komedogene Substanzen mindestens ein alkyl- oder hydroxyalkylsubstituierter Harnstoff der allgemeinen Formel (UREA) enthalten ist, 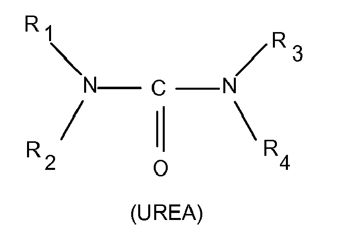 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Weitere bevorzugte erfindungsgemäße Zusammensetzungen sind **dadurch gekennzeichnet, dass** der mindestens eine alkyl- oder hydroxyalkylsubstituierte Harnstoff der allgemeinen Formel (UREA) ausgewählt ist aus Verbindungen, in denen R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-HydroxyalkylGruppen substituiert ist, stehen, mit der Maßgabe, dass mindestens einer der Reste R₁ und R₂ und gleichzeitig mindestens einer der Reste R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Weitere bevorzugte erfindungsgemäße Zusammensetzungen sind **dadurch gekennzeichnet, dass** der mindestens eine alkyl- oder hydroxyalkylsubstituierte Harnstoff der allgemeinen Formel (UREA) ausgewählt ist aus Bis-N,N'- (2-hydroxyethyl)harnstoff.

Erfindungsgemäß besonders bevorzugte Zusammensetzungen sind **dadurch gekennzeichnet, dass** sie mindestens einen alkyl- oder hydroxyalkylsubstituierten Harnstoff gemäß Formel (UREA), insbesondere N,N'-Bis-(2-hydroxyethyl)harnstoff, in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. N,N'-Bis-(2-hydroxyethyl)harnstoff, bei Raumtemperatur ein kristalliner Feststoff, ist beispielsweise als Handelsprodukt Hydrovance von AkzoNobel als ca. 45 - 55 %ige wässrige Lösung mit einem Gehalt an Harnstoff und Ammoniumlactat erhältlich.

In einer weiteren bevorzugten Ausführungsform enthalten die erfindungsgemäßen Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂CHOH-CH₂-COO⁻) jeweils mit Me = Methyl und X = C-C-Einfachbindung (im Falle des Betains) oder X = -CHOH-CH₂- (im Falle des Carnitins).

Erfindungsgemäß besonders bevorzugte Zusammensetzungen sind **dadurch gekennzeichnet, dass** sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen neben der erfindungsgemäßen Wirkstoffkombination mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Zusammensetzungen sind **dadurch gekennzeichnet, dass** sie neben der erfindungsgemäßen Wirkstoffkombination mindestens eine α-Hydroxycarbonsäure, α-Ketocarbonsäure und/oder β-Hydroxycarbonsäure oder ein Derivat, insbesondere einen Ester, ein Lacton oder ein Salz hiervon, in einer Gesamtmenge von 0,01 - 10 Gew.-%, bevorzugt 0,1 - 5 Gew.-%, besonders bevorzugt 0,5 - 1 - 2 Gew.-%, jeweils bezogen auf die gesamte Zusammensetzung, enthalten.

In einer weiteren bevorzugten Ausführungsform enthalten die erfindungsgemäßen Zusammensetzungen Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Erfindungsgemäß besonders bevorzugte Zusammensetzungen sind **dadurch gekennzeichnet, dass** sie Silymarin in Mengen von 0,00001 bis 1 Gew.-%, bevorzugt 0,0001 bis 0,01 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen Kreatin. Kreatin ist der Trivialname für *N*-Methyl-guanidino-essigsäure bzw. *N*-Amidinosarkosin. Erfindungsgemäß bevorzugt ist Kreatin in Mengen von 0,0001 bis 1 Gew.-%, besonders bevorzugt 0,001 bis 0,5 Gew.-% und außerordentlich bevorzugt 0,01 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens einen Wirkstoff, der ausgewählt ist aus den Mono- und Polyhydroxystilbenen und deren Estern. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert sein können. Mono- und Polyhydroxystilbene und deren Ester erhöhen und/oder verbessern die Interaktion zwischen der extrazellulären Matrix und den Fibroblasten. Erfindungsgemäß besonders bevorzugte Hydroxystilbene und deren Ester sind ausgewählt aus Resveratrol (trans-Stilben-3,4'-5-triol), den Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.

Erfindungsgemäß besonders bevorzugte kosmetische oder dermatologische Zusammensetzungen sind **dadurch gekennzeichnet, dass** sie mindestens einen Wirkstoff, der ausgewählt ist aus den Mono- und Polyhydroxystilbenen und deren Estern, in einer Gesamtmenge von 0,000001 - 5 Gew.-%, bevorzugt 0,00001 - 1 Gew.-%, besonders bevorzugt 0,0001 - 0,1 Gew.-% und außerordentlich bevorzugt 0,005 - 0,05 Gew.-%, enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens einen Desoxyzucker oder ein mindestens einen Desoxyzucker-Baustein enthaltendes Polysaccharid.

Erfindungsgemäß bevorzugte Desoxyzucker sind ausgewählt aus Rhamnose und Fucose. Ein erfindungsgemäß besonders bevorzugtes, Desoxyzucker-Bausteine enthaltendes Polysaccharid ist das mit der INCI-Bezeichnung Biosaccharide Gum-1, erhältlich als Handelsprodukt Fucogel^{®} von Solabia. Ein weiteres erfindungsgemäß besonders bevorzugtes, Desoxyzucker-Bausteine enthaltendes Polysaccharid ist das mit der INCI-Bezeichnung Biosaccharide Gum-2, erhältlich als Handelsprodukt Rhamnosoft^{®} von Solabia. Ein erfindungsgemäß besonders bevorzugtes, Desoxyzucker-Bausteine enthaltendes Polysaccharid ist das mit der INCI-Bezeichnung Biosaccharide Gum-3, erhältlich als Handelsprodukt Fucogenol^{®} von Solabia. Ein erfindungsgemäß besonders bevorzugtes, Desoxyzucker-Bausteine enthaltendes Polysaccharid ist das mit der INCI-Bezeichnung Biosaccharide Gum-4, erhältlich als Handelsprodukt Glycofilm^{®} von Solabia. Erfindungsgemäß bevorzugt sind weiterhin Mischungen der vorgenannten, mindestens einen Desoxyzucker-Baustein enthaltenden Polysaccharide, beispielsweise die Mischung aus Biosaccharide Gum-2 und Biosaccharide Gum-3, erhältlich als Handelsprodukt Elastinol plus^{®} von Solabia. Erfindungsgemäß besonders bevorzugte kosmetische oder dermatologische Zusammensetzungen sind **dadurch gekennzeichnet, dass** sie mindestens einen Desoxyzucker und/ oder mindestens ein Desoxyzucker-Bausteine enthaltendes Polysaccharid in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte erfindungsgemäße Zusammensetzung, enthalten.

Vorteilhafterweise liegen die erfindungsgemäßen Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

Ein weiterer Gegenstand der vorliegenden Erfindung ist die Verwendung von erfindungsgemäßen Zusammensetzungen zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut.

Bezüglich weiterer bevorzugter Ausführungsformen der erfindungsgemäßen Verwendungen gilt mutatis mutandis das zu den erfindungsgemäßen Mitteln Gesagte.

Ein weiterer Gegenstand der vorliegenden Erfindung ist ein nicht-therapeutisches Verfahren zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut, **dadurch gekennzeichnet, dass** ein erfindungsgemäßes Hautbehandlungsmittel auf die Haut aufgetragen wird.

Auch bezüglich weiterer bevorzugter Ausführungsformen der erfindungsgemäßen Verfahren gilt mutatis mutandis das zu den erfindungsgemäßen Mitteln Gesagte.

Es wurden folgende Hautcremes hergestellt (Angaben in Gew.-%):
1. Beispielserie:

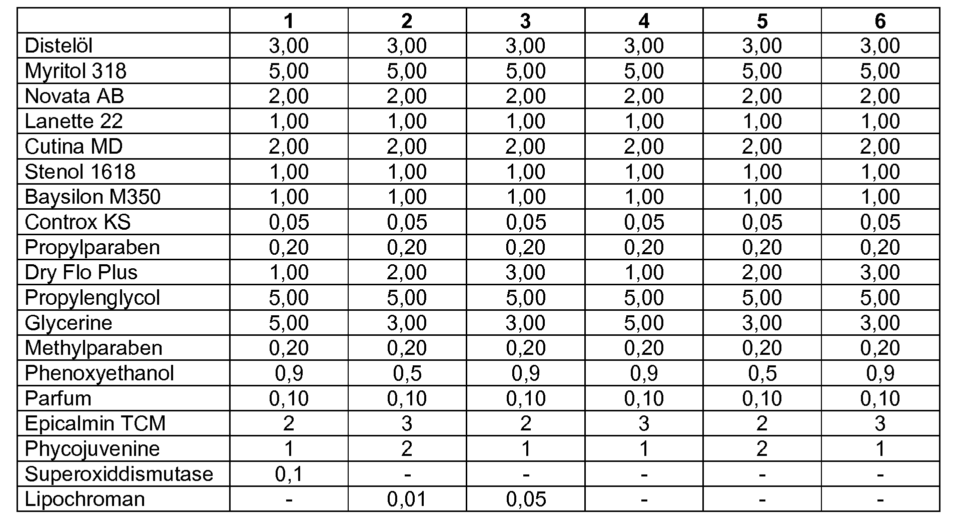

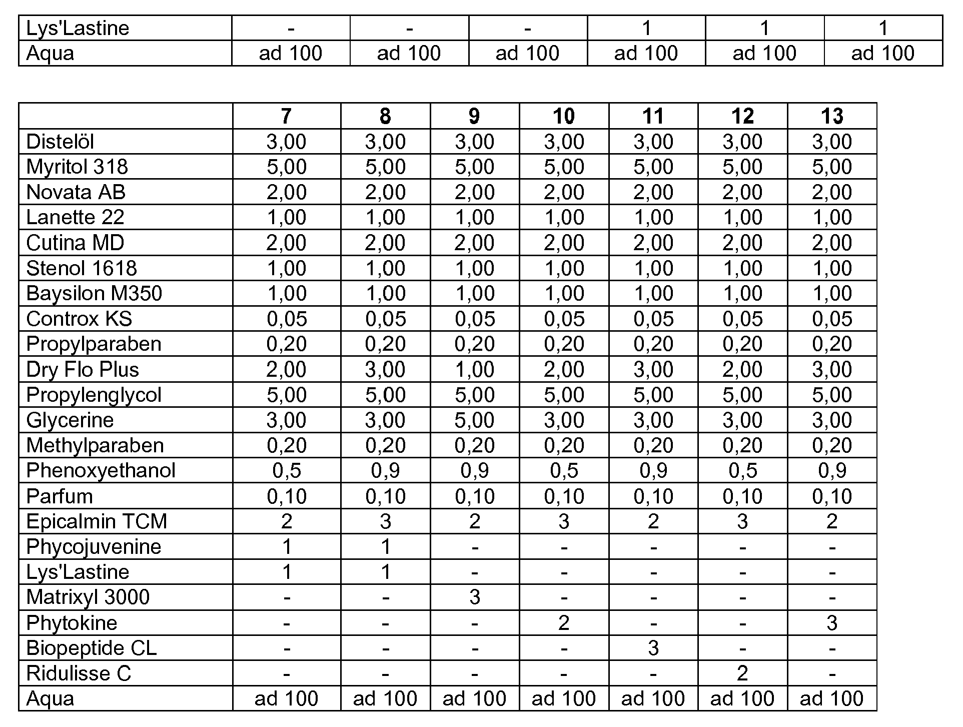

| | | |
|---|---|---|
| Myritol 318 | Caprylic/Capric Triglyceride | Cognis |
| Novata AB | Cocoglycerides | Cognis |
| LANETTE® 22 | Behenyl Alcohol | Cognis |
| Cutina MD | Glyceryl Stearate | Cognis |
| Stenol 16/18 | Cetearylalkohol | Cognis |
| Baysilone-Öl M 350 | Dimethicone | GE Bayer Silicones |
| Controx KS | Tocopherol, Hydrogenated Palm Glycerides Citrate | Cognis |
| Dry Flo Plus | Aluminium Starch Octenylsuccinate | National Starch |
| Epicalmin TCM | INCI: Lonicera Japonica Flower Extract/Lonicera Japonica (Honeysuckle) Flower Extract (and) Xanthium Sibiricum Fruit Extract (and) Cyperus Rotundus Root Extract (and) Sodium Benzoate (and) Aqua/Water | Mibelle Biochemistry |
| Phycojuvenine | Laminaria Digitata Extract, ca. 3%ig in Wasser | Codif |
| Lipochroman | DIMETHYLMETHOXY CHROMANOL | Lipotec |
| Lys'Lastine | Dillextrakt, 0,2-3%ig in Wasser/Butylenglycol; INCI-Bezeichnung: Aqua (Water), Butylene Glycol, Peucedanum Graveolens (Dill) Extract, Xanthan Gum | BASF |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Phytokine | HYDROLYZED SOY PROTEIN | Coletica |
| Ridulisse C | HYDROLYZED SOY PROTEIN | Silab |
| Biopeptide CL | N-Palmitoyl-Gly-His-Lys | Sederma |

2. Beispielserie:

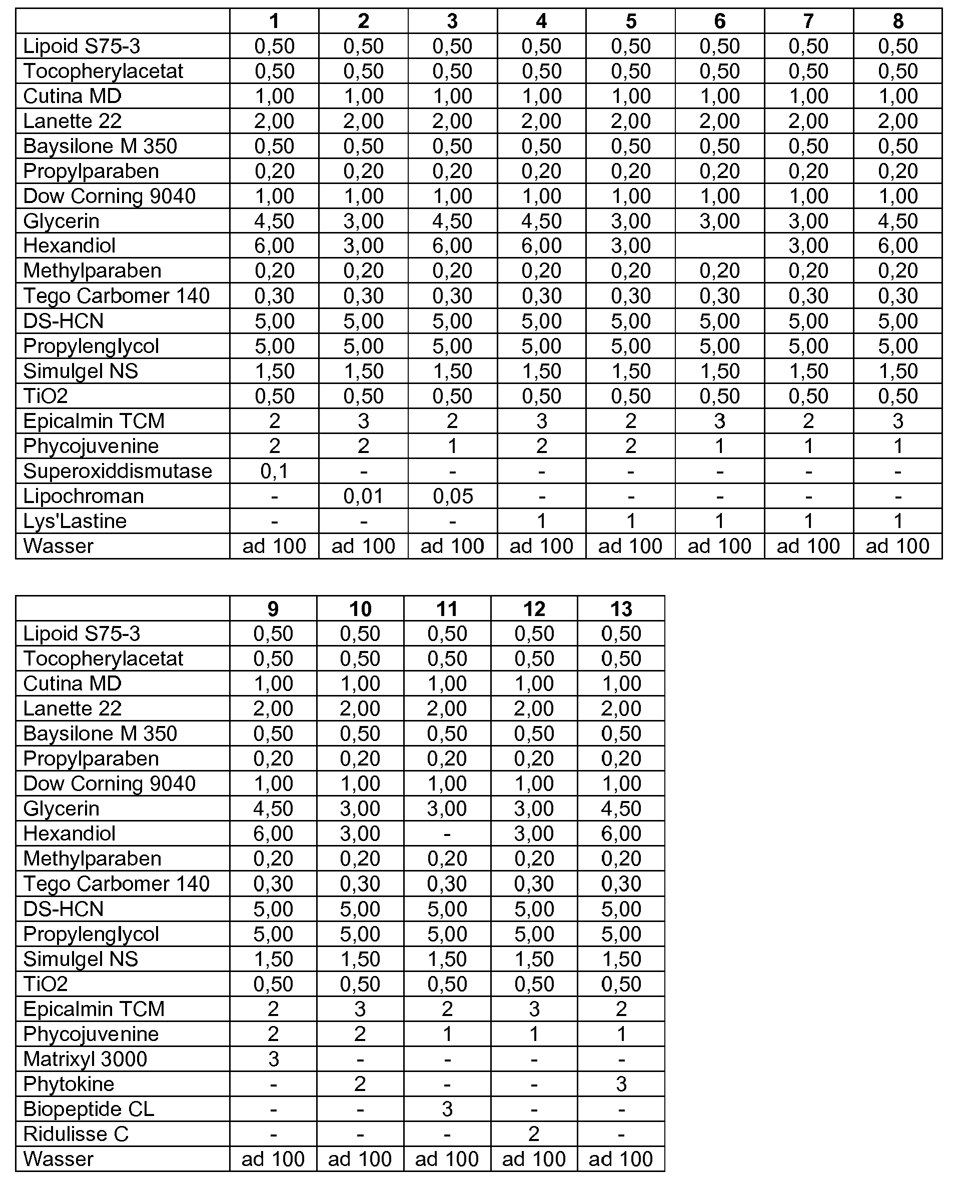

| | | |
|---|---|---|
| Lipoid S 75-3 | Hydrogenated Lecithin | Lipoid GmbH |
| Dow Corning 9040 | Cyclomethicone/ Dimethicone Crosspolymer | Dow Corning |
| Tego Carbomer^{®} 140 | Carboxyvinylpolymer (INCI-Bezeichnung: Carbomer) | Goldschmidt |
| DSH CN | Water, Dimethylsilanol Hyaluronate | Exsymol |
| Simulgel NS | Hydroxyethyl Acrylate / Sodium Acryloyl-dimethyl Taurate Copolymer / Squalane / Polysorbate 60 | Seppic |

3. Beispielserie:

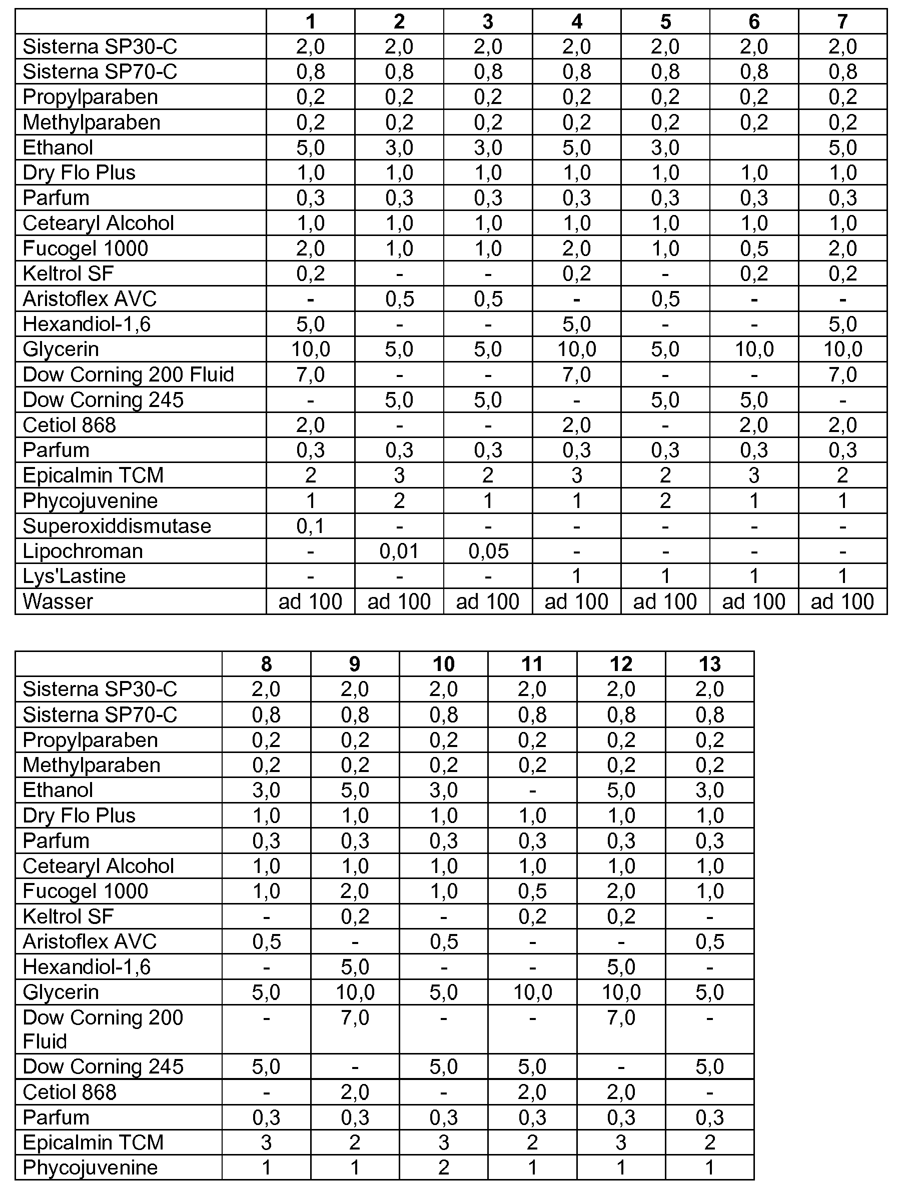

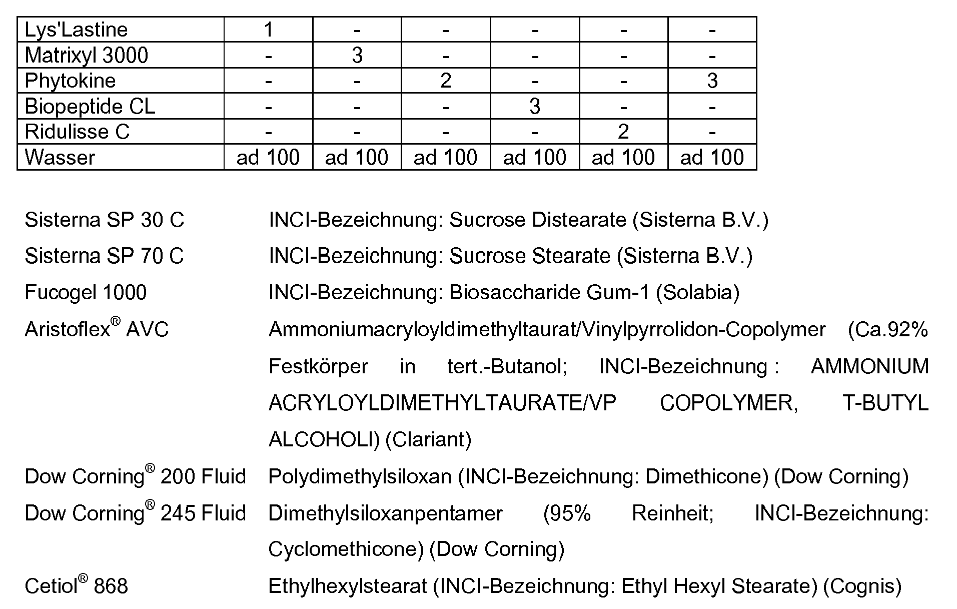

4. Beispielserie:

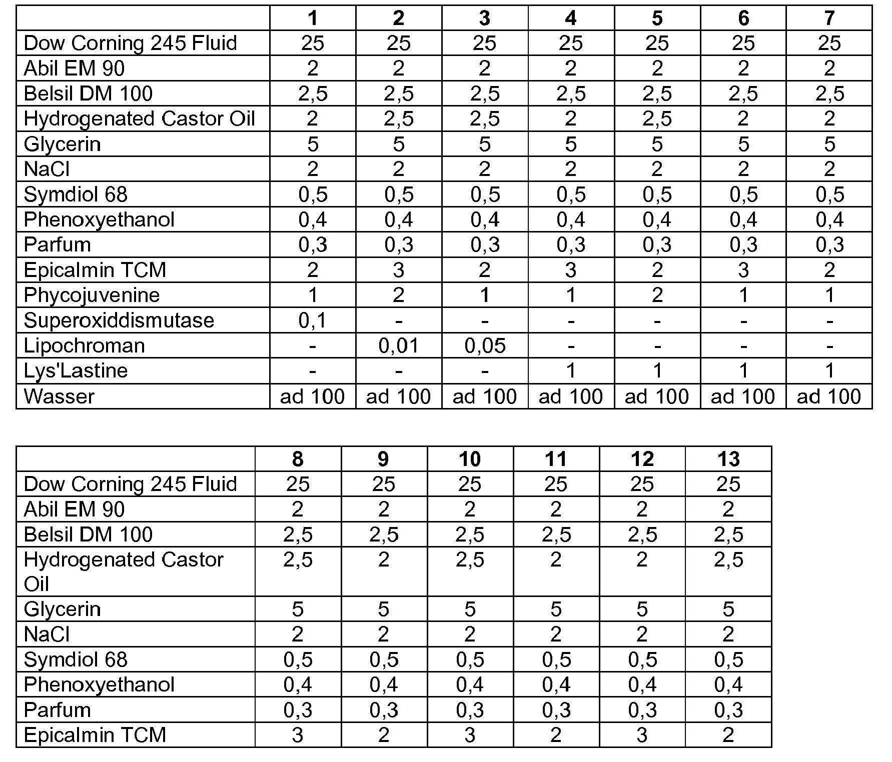

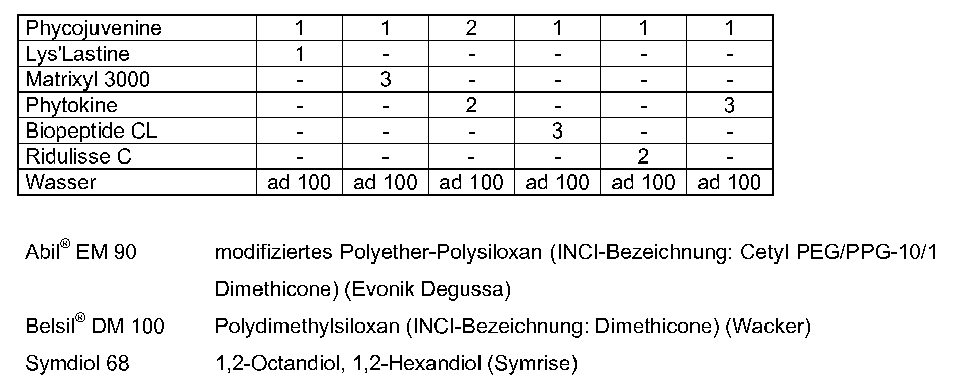

5. Beispielserie:

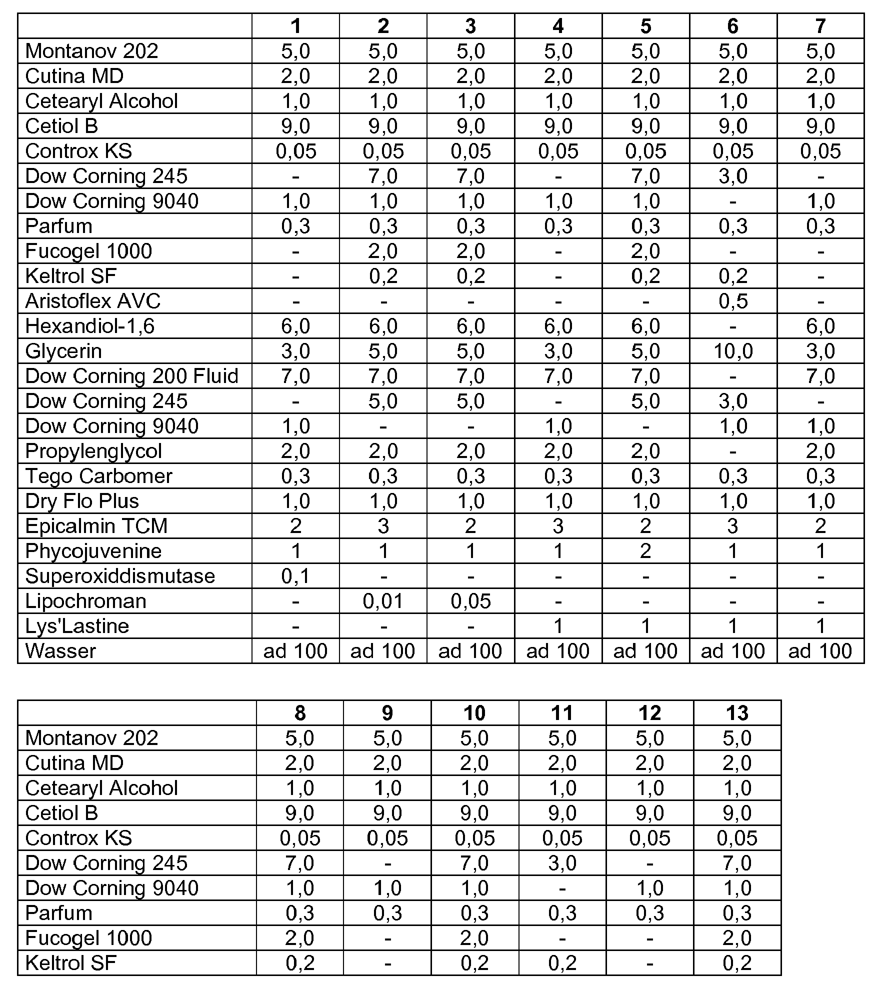

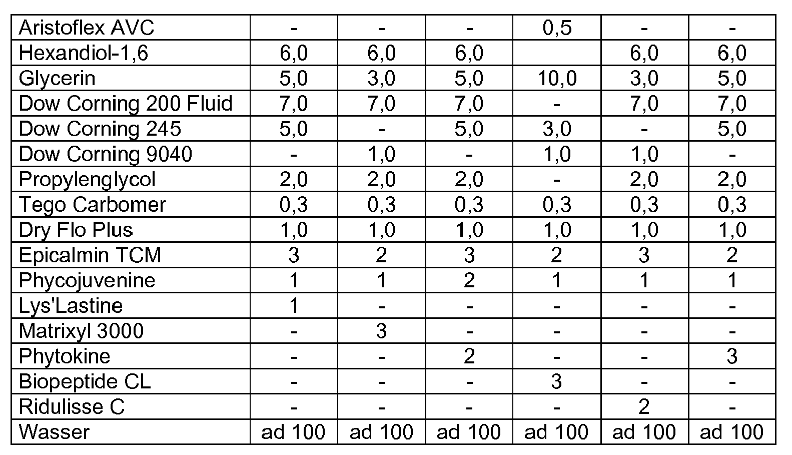

6. Beispielserie:

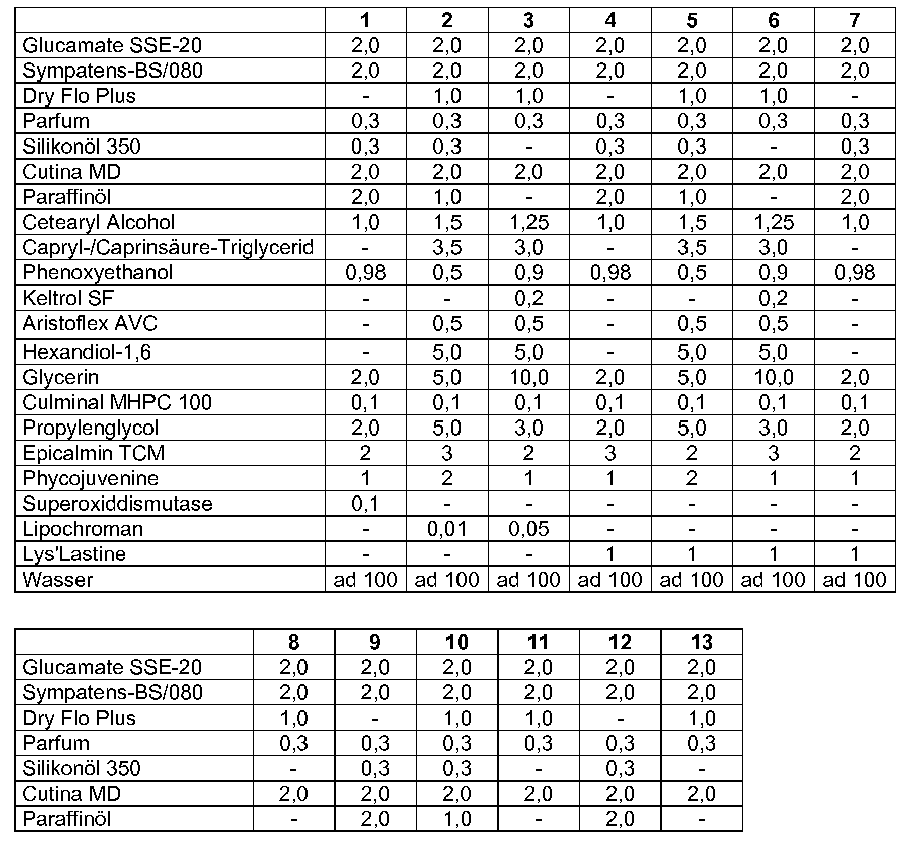

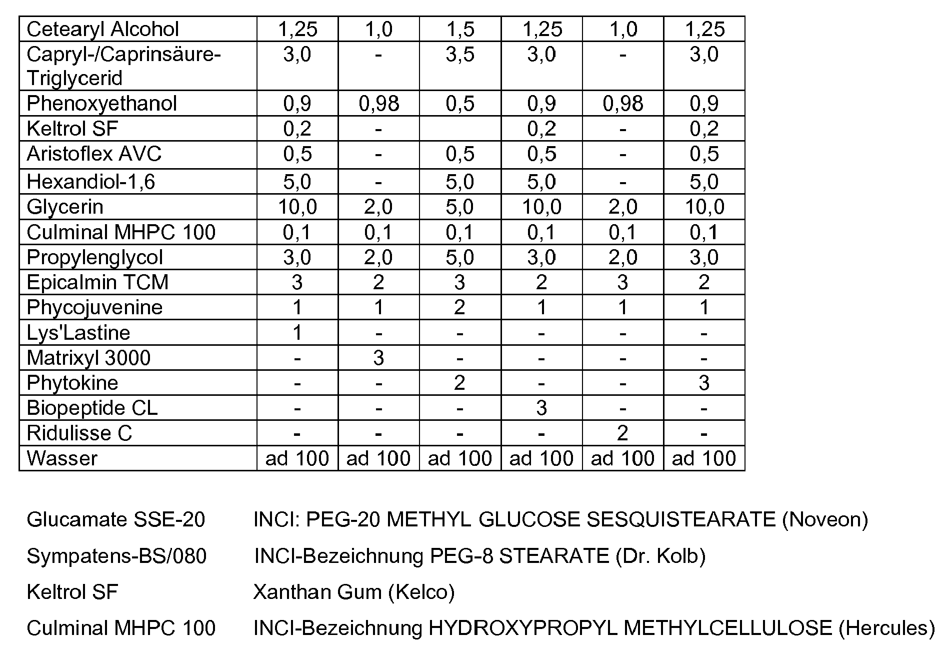

7. Beispielserie:

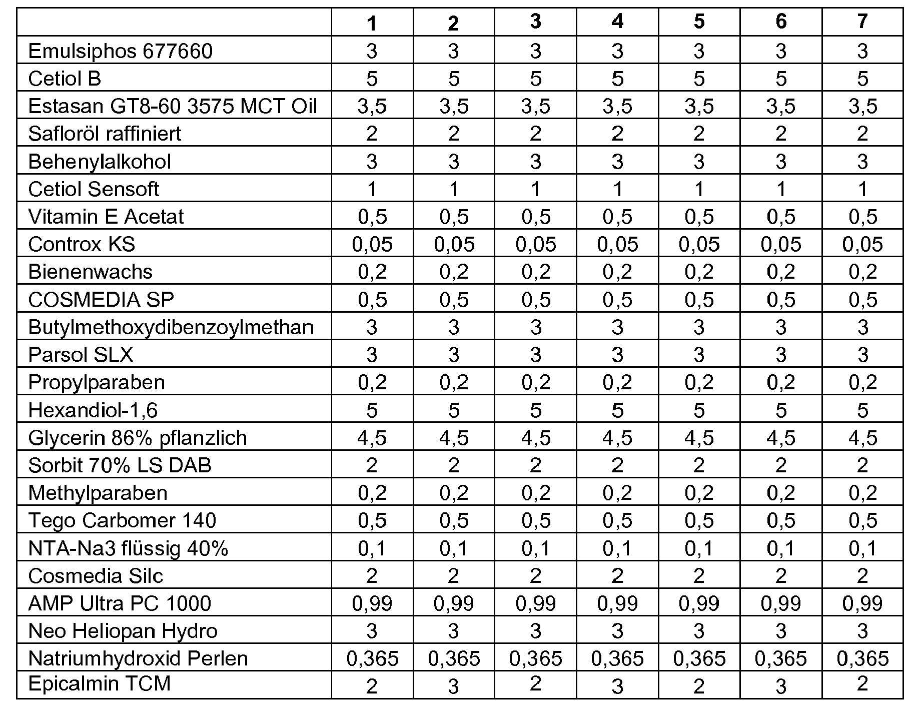

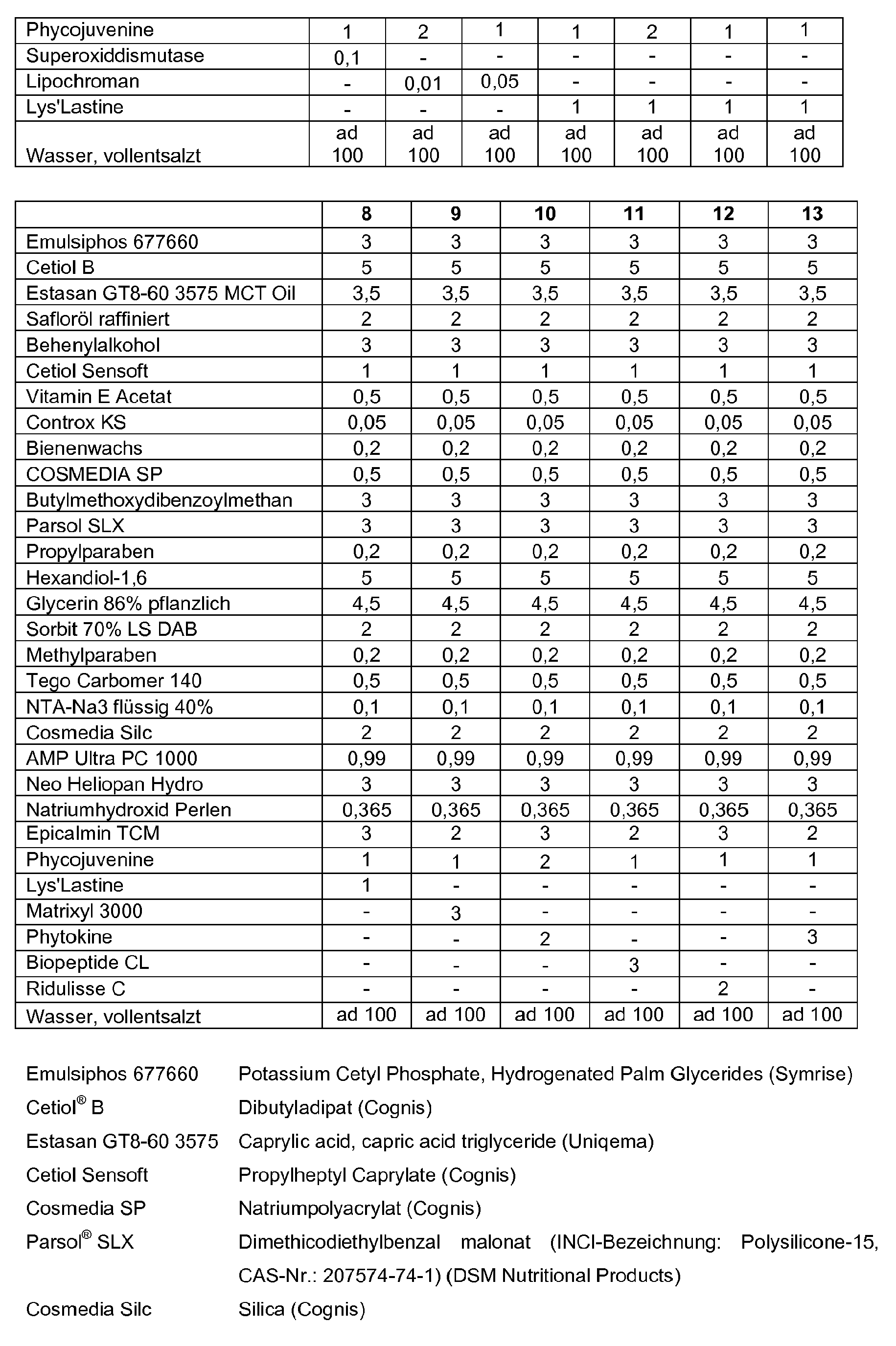

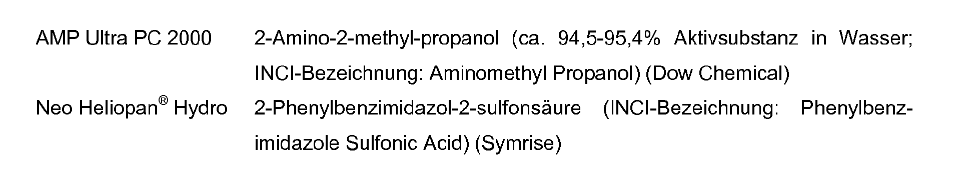

## Patentansprüche

1. Kosmetische oder dermatologische topische Zusammensetzungen, enthaltend in einem geeigneten kosmetischen oder dermatologischen Träger - bezogen auf ihr Gewicht -
a) mindestens einen Extrakt aus den Blüten von Lonicera japonica,
b) mindestens einen Extrakt aus den Früchten von Xanthium sibiricum,
c) mindestens einen Extrakt aus den Wurzeln von Cyperus rotundus,
d) 0,001 bis 10 Gew.-% mindestens eines Wirkstoffes aus der Gruppe
- der Monomeren, Oligomeren und Polymeren von Aminosäuren, N-C₂-C₂₄-Acylaminosäuren, den Estern und/oder den physiologisch verträglichen Metallsalzen dieser Substanzen und/oder
- der Extrakte aus der Gruppe, bestehend aus Dill (Peucedanum Graveolens), Korinthe (Johannisbeere), Kardamom (Elettaria cardamomum), schwarzem Rettich, kleiner Stechpalme, Zimt, Hafer (Avena sativa), Kartoffel, Seide, und Asea foetida gum und/oder
- Hyaluronsäure und/oder
- Apfelkernextrakt und/oder
- der Phytohormone und/oder
- der Isoflavonoide und/oder
- der Phytosterole und/oder
- der Triterpenoide und/oder
- der Tocopherole
- der 6,7-disubstituierten 2,2-Dialkylchromanen oder -chromenen der allgemeinen
Formeln (II) oder (III), 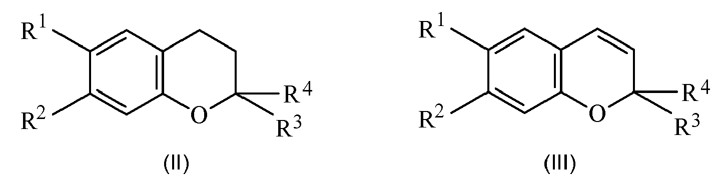 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, insbesondere Lipochroman-6 und/oder
- Ellagsäure und/oder Ellagate und/oder
- der Xanthophylle, insbesondere Astaxanthin, und/oder
- der Superoxiddismutasen und/oder
- Milchsäurebakterien-basierten Fermentationen und/oder
- Ethylhexenoat und seinen Derivaten und/oder
- Methylbutyrat und seinen Derivaten und/oder
- Ethyldecadienoat und seinen Derivaten.

2. Zusammensetzung gemäß Anspruch 1, **dadurch gekennzeichnet, dass** sie - bezogen auf ihr Gewicht - 0,001 bis 10 Gew.-%, vorzugsweise 0,002 bis 9 Gew.-%, weiter bevorzugt 0,005 bis 8 Gew.-%, noch weiter bevorzugt 0,01 bis 7 Gew.-% und insbesondere 0,05 bis 6 Gew.-% Extrakt(e) aus den Blüten von Lonicera japonica enthält.

3. Zusammensetzung nach einem der Ansprüche 1 oder 2, **dadurch gekennzeichnet, dass** sie - bezogen auf ihr Gewicht - 0,001 bis 10 Gew.-%, vorzugsweise 0,002 bis 9 Gew.-%, weiter bevorzugt 0,005 bis 8 Gew.-%, noch weiter bevorzugt 0,01 bis 7 Gew.-% und insbesondere 0,05 bis 6 Gew.-% Extrakt(e) aus den Früchten von Xanthium sibiricum enthält.

4. Zusammensetzung nach einem der Ansprüche 1 bis 3, **dadurch gekennzeichnet, dass** sie - bezogen auf ihr Gewicht - 0,001 bis 10 Gew.-%, vorzugsweise 0,002 bis 9 Gew.-%, weiter bevorzugt 0,005 bis 8 Gew.-%, noch weiter bevorzugt 0,01 bis 7 Gew.-% und insbesondere 0,05 bis 6 Gew.-% Extrakt(e) aus den Wurzeln von Cyperus rotundus enthält.

5. Zusammensetzung nach einem der Ansprüche 1 bis 4, **dadurch gekennzeichnet, dass** sie die Monomeren von Aminosäuren, N-C₂-C₂₄-Acylaminosäuren und/oder den physiologisch verträglichen Metallsalzen dieser Substanzen ausgewählt sind aus Alanin, Arginin, Asparagin, Asparaginsäure, Canavanin, Citrullin, Cystein, Cystin, Desmosin, Dipalmitoylhydroxyprolin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat.

6. Zusammensetzung nach einem der Ansprüche 1 bis 5, **dadurch gekennzeichnet, dass** die Oligomeren von Aminosäuren, N-C₂-C₂₄-Acylaminosäuren und/oder den physiologisch verträglichen Metallsalzen dieser Substanzen ausgewählt sind aus Di-, Tri-, Tetra-, Penta-, Hexa- oder Pentadecapeptiden, die N-acyliert und/oder verestert sein können.

7. Zusammensetzung nach Anspruch 6, **dadurch gekennzeichnet, dass** das Oligopeptid ausgewählt ist aus Tyr-Arg und seinen N-acylierten Derivaten, insbesondere N-Acetyl-Tyr-Arg-hexyldecylester, Gly-His-Lys und seinen N-acylierten Derivaten, insbesondere N-Palmitoyl-Gly-His-Lys, Gly-His-Arg, und seinen N-acylierten Derivaten, insbesondere N-Myristoyl-Gly-His-Arg, Lys-Val-Lys und seinen N-acylierten Derivaten, insbesondere Palmitoyl-Lys-Val-Lys, Val-Tyr-Val, Gly-Gln-Pro-Arg (Rigin), Rigin-Analoga und Rigin-Derivaten, insbesondere N-Palmitoyl-Gly-Gln-Pro-Arg, Lys-Thr-Thr-Lys-Ser und seinen N-acylierten Derivaten, insbesondere N-Palmitoyl-Lys-Thr-Thr-Lys-Ser, Val-Gly-Val-Ala-Pro-Gly und seinen N-acylierten Derivaten, insbesondere N-Palmitoyl-Val-Gly-Val-Ala-Pro-Gly, Acetyl-Hexapeptide-3, Hexapeptide-4, Hexapeptide-5, Myristoyl Hexapeptide-5, Myristoyl Hexapeptide-6, Hexapeptide-8, Hexapeptide-9, Hexapeptide-10, L-Glutamylaminoethyl-indol, sowie Kombinationen dieser Substanzen, insbesondere Kombinationen aus N-Palmitoyl-Gly-His-Lys und N-Palmitoyl-Gly-Gln-Pro-Arg.

8. Zusammensetzung nach einem der Ansprüche 1 bis 7, **dadurch gekennzeichnet, dass** die Polymeren von Aminosäuren, N-C₂-C₂₄-Acylaminosäuren und/oder den physiologisch verträglichen Metallsalzen dieser Substanzen ausgewählt sind aus tierischen und pflanzlichen Proteinhydrolysaten, tierischen und pflanzlichen Proteinen und DNA-Reparaturenzymen.

9. Zusammensetzung nach einem der Ansprüche 1 bis 8, **dadurch gekennzeichnet, dass** sie - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% 6,7-disubstituierte 2,2-Dialkylchromane oder -chromene der allgemeinen Formeln (II) oder (III) wie vorstehend definiert, enthält, wobei bevorzugte Zusammensetzungen - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Lipochroman-6 enthalten.

10. Zusammensetzung nach einem der Ansprüche 1 bis 9, **dadurch gekennzeichnet, dass** sie - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Ellagsäure (2,3,7,8-Tetrahydroxy[1]benzopyrano-[5,4,3-*cde*][1]benzopyran-5,10-dion) und/oder Ellagate enthält.

11. Zusammensetzung nach einem der Ansprüche 1 bis 10, **dadurch gekennzeichnet, dass** sie - bezogen auf ihr Gewicht - 0,001 bis 10 Gew.-%, vorzugsweise 0,005 bis 7,5 Gew.-%, weiter bevorzugt 0,01 bis 5 Gew.-% und insbesondere 0,02 bis 1 Gew.-% Xanthophylle, vorzugsweise Canthaxanthin, Citranaxanthin, Flavoxanthin, Rhodoxanthin, Rubixanthin, Zeaxanthin und ganz besonders bevorzugt Astaxanthin, enthält.

12. Zusammensetzung nach einem der Ansprüche 1 bis 11, **dadurch gekennzeichnet, dass** sie - bezogen auf ihr Gewicht - 0,001 bis 10 Gew.-%, vorzugsweise 0,005 bis 7,5 Gew.-%, weiter bevorzugt 0,01 bis 5 Gew.-% und insbesondere 0,02 bis 2 Gew.-% Superoxiddismutase(n) enthält.

13. Zusammensetzung nach einem der Ansprüche 1 bis 12, **dadurch gekennzeichnet, dass** sie zusätzlich mindestens ein DNA-Reparaturenzym, vorzugsweise Photolyase und/oder T4 Endonuclease V und/oder 8-Oxoguanin-glycosylase und/oder deren Mischungen enthält.

14. Verwendung einer Zusammensetzung nach einem der Ansprüche 1 bis 13 zur nicht-therapeutischen, kosmetischen Behandlung und/oder Minimierung von Hautfalten und - fältchen, den Anzeichen der intrinsischen und extrinsischen Hautalterung, müder und/oder schlaffer Haut, UV-geschädigter Haut und/oder gereizter Haut.

15. Nicht-therapeutisches Verfahren zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut, **dadurch gekennzeichnet, dass** ein Hautbehandlungsmittel gemäß einem der Ansprüche 1 bis 13 auf die Haut aufgetragen wird.
